Europäisches Patentamt

European Patent Office

Office européen des brevets

(11) Publication number: **0 125 725**
**B1**

## EUROPEAN PATENT SPECIFICATION

(45) Date of publication of patent specification: **16.06.87**

(21) Application number: **84200652.0**

(22) Date of filing: **08.05.84**

(51) Int. Cl.⁴: **A 61 K 9/08, A 61 K 31/65**

(54) **Oxytetracyclin solutions.**

(30) Priority: **09.05.83 NL 8301633**

(43) Date of publication of application:
**21.11.84 Bulletin 84/47**

(45) Publication of the grant of the patent:
**16.06.87 Bulletin 87/25**

(84) Designated Contracting States:
**AT BE CH DE FR GB IT LI LU NL SE**

(56) References cited:
**EP-A-0 012 495**
**GB-A-2 047 097**
**GB-A-2 063 669**
**US-A-3 957 972**

(73) Proprietor: **Gist - Brocades N.V.**
**Wateringseweg 1 P.O. Box 1**
**NL-2600 MA Delft (NL)**

(72) Inventor: **Tan, Hong Sheng**
**Buizerdstraat 94**
**NL-2665 TG Bleiswijk (NL)**
Inventor: **Dijkgraaf, Bernardus Leonardus J.**
**Van der Haertstraat 9**
**NL-2613 XZ Delft (NL)**
Inventor: **Beukers, Robert**
**Sijtwinde 159**
**NL-2631 GZ Nootdorp (NL)**

(74) Representative: **Schmieman, Johannes Hendrik et al**
**Gist-Brocades N.V. Patents & Trademarks Department Martinus Nijhofflaan 2 P.O. Box 1**
**NL-2600 MA Delft (NL)**

Courier Press, Leamington Spa, England.

# 0 125 725

**Description**

The invention relates to injectable solutions of oxytetracycline and their preparation. Oxytetracycline (OTC) as such is not stable in aqueous solution. Moreover, it is poorly soluble in water. Acceptable solutions can be obtained by addition of certain excipients. It is known, for instance, that by addition of magnesium compounds a magnesium complex is formed which dissolves better than oxytetracycline itself. It is further known to improve the solubility by addition of polyvinylpyrrolidone (PVP) and/or organic solvents. Preparations with magnesium and PVP, as described in Dutch patent 134511, are found to be very satisfactory in practice. Their only disadvantage is that the oxytetracycline content is restricted by the limitation which is put in practice to the viscosity. With a content of 10% or more the viscosity increases to such an extent that the application is hampered.

There is a need, however, for preparations with a higher concentration of oxytetracycline for veterinary purposes. In the first place because one prefers to keep the injection volume as small as possible (particularly important for animals with a weight of more than 100 kg) and second because it has been found that when using higher dosages with more concentrated solutions a considerable longer therapeutical effect is achieved.

Up till now it was only possible to prepare solutions with higher oxytetracycline concentration with low viscosity by using certain organic solvents such as propylene glycol, dimethylacetamide, 2-pyrrolidone, polyethylene glycol and N-methylpyrrolidone, to which solvents sometimes PVP also was added. Such solutions are described in British Patent 1,427,882, British Patent Application 2047097, Dutch patent application 7804455, Belgian patent 861885, German patent applications 2001604, 2501805 and 3104233 and U.S. patent 4,018,889. All the solvents used have the strong disadvantage that they cause a strong tissue irritation on the site of the injection, which even can lead to visible abnormalities in the meat in this place with an accompanying loss of value.

When PVP is applied, a type is preferably chosen with a mean molecular weight between 10,000 and 40,000.

It has now been found that one can reach higher concentrations of oxytetracyclin with a good stability, an acceptable viscosity and a minimal tissue irritation by using PVP with a relatively low molecular weight.

The use of low molecular PVP has been disclosed in European patent application published under No. 0012495 and in British patent 2063669, directed to dry preparations of amoxycillin and dicloxacillin respectively, which are intended to be reconstituted with water before use.

US patent 3,957,972 discloses an aqueous solution of 1—5% by weight of OTC, containing 2.5—7.49% PVP having a molecular weight in the range of 3000 to 60,000. However, no actual experience is disclosed with PVP of a molecular weight lower than 10,000 (the lowest molecular weight medical grade PVP actually available at the date of that patent).

The invention comprises aqueous injectable solutions containing oxytetracycline, a magnesium compound and polyvinylpyrrolidone as the solubilizing agent, with a pH of 8—9.5, characterized in that the polyvinylpyrrolidone has a molecular weight between 500 and 10,000, and is present in a concentration of at least 18% (W/V), and the oxytetracycline is present in a concentration of at least 10% (W/V).

The PVP concentration is at least 18% (w/v) because solutions can thus be obtained which contain 10 or more percent by weight of oxytetracycline. According to the invention, solutions can be obtained with OTC up to 20% (w/v). For that, maximally 40% (w/v) of PVP is needed. The kind and the amount of magnesium compound can be the same as described in Dutch patent specification 134,511. Magnesium oxide and magnesium chloride aq are for instance suitable.

The invention also includes the preparation of oxytetracycline solutions by dissolving in water oxytetracycline as salt or as a base with an appropriate amount of a magnesium compound and PVP and bringing the pH in the range of 8.0 to 9.5, characterized in that the PVP has a molecular weight between 500 and 10,000 and a final concentration of at least 18% (w/v) and the oxytetracycline is present in a final concentration of at least 10% (w/v). The amount of magnesium compound to be used is preferable 1 to 1.5 mol per mol of oxytetracycline. The pH can be brought to the desired value with the aid of a physological harmless base, such as ammonia, ethylenediamine or ethanolamine.

In order to promote the stability, the air in bottles or ampoules containing the oxytetracycline solutions of the invention can be replaced by an inert gas, preferably nitrogen. It is also advantageous for the stability to add to the solution a small amount of a reducing agent, such as sodium metabisulfite, sodium sulfite or sodium formaldehyde-sulfoxylate.

It was conceivable that substitution of the PVP commonly used in known preparations by PVP of lower molecular weight would lead to tissue irritation. Surprisingly this is not the case. The effect of lowering the molecular weight of PVP on the stability of the injection solution and the level of oxytetracycline in the blood could not be predicted. It is therefore unobvious that the compositions of the invention possess a satisfactory stability and at higher dosages show a long lasting therapeutic blood level of the oxytetracycline.

An accompanying advantage of the use of low molecular PVP is that the likelihood of tumours to be formed, as seen with PVP with higher molecular weight, will be minimal.

The preparation of the oxytetracycline-solutions according to the invention is illustrated by the following examples in which the percentages mentioned are weight by volume percents.

2

**0 125 725**

Example 1
Preparation of a 10% oxytetracycline solution with 18% of polyvinylpyrrolidone (K-value 11—14)
Under a nitrogen atmosphere the following was added successively to 200 ml of pyrogen free water:

90 g of polyvinylpyrrolidone (K-value 11—14, molecular weight 2000—3500),
3 g of sodium formaldehydesulfoxylate,
4.34 g of magnesium oxide,
11 g of ethanolamine, and a solution of 56.7 g of oxytetracycline hydrochloride in 100 ml of pyrogen free water.

After being stirred for some time, a clear solution was formed. The solution was made up to 500 ml with pyrogen free water, which gave a solution with a pH of 8.7 and a viscosity of 17 milli Pascal second (m Pas) at 22°C. The solution contained 100 mg of oxytetracycline per ml. This solution was filtered through a sterilizing filter, after which stability studies were carried out. At 50°C the solution was stable for 3 to 4 weeks, but at 37°C the solution remained stable even after 6 months. As a test for the stability of the solution the absence of a precipitate was used.

Example 2
Preparation of a 10% oxytetracycline solution with 20% of polyvinylpyrrolidone (K-value 11—14)
Under a nitrogen atmosphere the following was added successively to 200 ml of pyrogen free water:
100 g of polyvinylpyrrolidone (K-value 11—14), molecular weight 2000—3500),
3 g of sodium formaldehydesulfoxylate,
4.34 g of magnesium oxide,
11 g of ethanolamine and a solution of 56.7 g of oxytetracycline hydrochloride in 100 ml of pyrogen free water.

After being stirred for some time a clear solution was formed which was made up to 500 ml with pyrogen free water. The solution obtained contained 100 mg of oxytetracycline per ml and had a pH of 8.8 and a viscosity of 21 m Pas at 22°C. After sterile filtration the solution remained stable for about 8 weeks at 50°C and at 37°C the stability was more than 6 months. After 6 months at 37°C the solution kept its light colour.

Example 3
Preparation of a 10% oxytetracycline-solution with 20% of polyvinylpyrrolidone (K-value 11—14) starting from oxytetracycline base
a. Preparation of oxytetracycline base
60 g of oxytetracycline hydrochloride was dissolved in 800 ml of boiled pyrogen free water. With the aid of 4 N sodium hydroxide, which also was prepared with boiled pyrogen free water, the pH was brought to 5.35 under stirring, which made the oxytetracycline base precipitate. The precipitate was filtered off and washed thoroughly with pyrogen free water.

b. Preparation of the oxytetracycline/polyvinylpyrrolidone solution
Under a nitrogen atmosphere the following was added successively to 200 ml of pyrogen free water:
100 g of polyvinylpyrrolidone (K-value 11—14) molecular weight 2000—3500),
2.5 g of sodium formaldehydesulfoxylate,
4.34 g of magnesium oxide, and the oxytetracycline base obtained by procedure a. With the aid of ethanolamine the pH was brought to 8.5 to 9.0. A clear solution was formed. Pyrogen free water was added to 500 ml and then the solution was filtered through a sterilizing filter. The solution obtained contained 100 mg of oxytetracycline per ml, had a pH of 8.9 and a viscosity of 22 m Pas at 22°C. The solution remained stable at 50°C for more than 8 weeks and at 37°C for more than 6 months.

Example 4
Preparation of a 10% oxytetracycline solution with 25% of polyvinylpyrrolidone (K-value 11—14)
In the same way as in Example 2 a solution was made which contained 250 mg of polyvinylpyrrolidone per ml. However, instead of 100 g of polyvinylpyrrolidone (K-value 11—14) 125 g were added. The sterile filtered solution obtained had a pH of 8.7 and a viscosity of 31 m Pas at 22°C. The stability at 50°C was more than 8 weeks, but the solution took on a dark colour. At 37°C the solution still had a light colour after 6 months and no precipitate had been formed.

Example 5
Preparation of a 10% oxytetracycline solution with 30% of polyvinylpyrrolidone (K-value 11—14)
The preparation was carried out in the same way as in Example 2. However, instead of 100 g of polyvinylpyrrolidone with a K-value of 11—14, 150 g were used. After filtration through a sterilizing filter the solution had a pH of 8.7 and a viscosity of 48 m Pas at 22°C. The stability at 50°C was more than 8 weeks and at 37°C more than 6 months.

3

Example 6
Preparation of a 10% oxytetracycline solution with 30% of polyvinylpyrrolidone (K-value 11—14) starting from oxytetracycline base

In the same way as described in Example 3 under a the base was prepared. The procedure was then continued as described in Example 3 under b, except that instead of 100 g of polyvinylpyrrolidone (K-value 11—14) 150 g were used. After filtration through a sterilizing filter, a solution was obtained which contained 100 mg of oxytetracycline per ml. The pH was 8.8 and the viscosity 43 m Pas at 22°C. At 50°C the solution remained clear for more than 8 weeks and at 37°C for more than 6 months.

Example 7
Preparation of a 10% oxytetracycline solution with 40% of polyvinylpyrrolidone (K-value 11—14)

The preparation was identical to that described in Example 2, except that instead of 100 g of polyvinylpyrrolidone with a K-value of 11—14, 200 g were used. After filtration through a sterilizing filter, the solution had a pH of 8.9 and a viscosity of 66 m Pas at 22°C. The solution remained stable at 50°C for more than 8 weeks and at 37°C for more than 6 months.

Example 8
Preparation of a 10% oxytetracycline solution with 40%. of polyvinylpyrrolidone (K-value 11—14) starting from oxytetracycline base

The preparation was identical to that described in Example 3, except that instead 100 g of polyvinylpyrrolidone with a K-value of 11—14, 200 g were used. After filtration through a sterilizing filter, the solution had a pH of 8.9 and a viscosity of 100 m Pas at 20°C. At 50°C the solution remained stable for more than 8 weeks and at 37°C for more than 6 months.

Example 9
Preparation of a 15% oxytetracycline solution with 20% of polyvinylpyrrolidone (K-value 11—14)

The following was added successively under a nitrogen atmosphere to 200 ml of boiled pyrogen free water:

100 g of polyvinylpyrrolidone,
2.5 g of sodium formaldehydesulfoxylate,
6.5 g of magnesium oxide,
85.1 g of oxytetracycline hydrochloride, and
17.9 g of ethanolamine.

The solution was made up to 500 ml and it was subsequently filtered through a sterilizing filter. The pH of the solution was 9.0 and the viscosity 80 m Pas at 22°C.

Example 10
Preparation of a 15% oxytetracycline solution with 20% of polyvinylpyrrolidone (K-value 11—14) starting from oxytetracycline base

Starting from 90 g of oxytetracycline hydrochloride, the base was first prepared.

The preparation was identical to the procedure described in Example 3 under a. Subsequently the following was added successively under a nitrogen atmosphere to 200 ml of pyrogen free water:

100 g of polyvinylpyrrolidone (K-value 11—14),
2.5 g of sodium formaldehydesulfoxylate,
6.5 g of magnesium oxide and the oxytetracycline base.

With the aid of ethanolamine the pH was brought to 8.5 to 9.0, after which the volume was made up to 500 ml. The clear solution was filtered through a sterilizing filter. A solution was obtained with a pH of 8.8 and a viscosity of 71 m Pas at 22°C.

Example 11
Preparation of a 15% oxytetracycline solution with 30% of polyvinylpyrrolidone (K-value 11—14)

Under a nitrogen atmosphere the following was added successively to 200 ml of boiled pyrogen free water:

150 g of polyvinylpyrrolidone (K-value 11—14),
2.5 g of sodium formaldehydesulfoxylate,
6.5 g of magnesium oxide,
85.1 g of oxytetracycline hydrochloride, and
17.9 g of ethanolamine.

The solution was made up to 500 ml with pyrogen free water and filtered through a sterilizing filter. A solution was obtained which contained 150 mg of oxytetracycline per ml. The solution had a pH of 9.0 and a viscosity of 180 m Pas at 22°C.

Example 12
Preparation of a 15% oxytetracycline solution with 30% of polyvinylpyrrolidone (K-value 11—14) starting from oxytetracycline base

Starting from 90 g of oxytetracycline hydrochloride the base was prepared in the manner described in

4

Example 3 under a. The following was added successively to 200 ml of pyrogen free water under a nitrogen atmosphere:

150 g of polyvinylpyrrolidone, (K-value 11—14),

2.5 g of sodium formaldehydesulfoxylate,

6.5 g of magnesium oxide, and the oxytetracycline base described above.

The volume was made up to 500 ml and the solution was filtered through a sterilizing filter. The solution obtained contained 150 mg of oxytetracycline per ml. The pH of the solution was 8.9 and the viscosity at 22°C was 120 m Pas.

Example 13

Preparation of a 20% oxytetracycline solution with 20% of polyvinylpyrrolidone (K-value 11—14)

The following was added successively under a nitrogen atmosphere to 200 ml of pyrogen free water:

100 g of polyvinylpyrrolidone (K-value 11—14),

2.5 g of sodium formaldehydesulfoxylate,

8.7 g of magnesium oxide,

113.4 g of oxytetracycline hydrochloride, and

23.8 g of ethanolamine.

The volume was made up to 500 ml with pyrogen free water. The solution obtained contained 200 mg of oxytetracycline per ml and had a pH of 9.0. The viscosity at 22°C was 425 mPas.

Example 14

Preparation of a 20% oxytetracycline solution with 30% of polyvinylpyrrolidone (K-value 11—14)

The following was successively added under a nitrogen atmosphere to 200 ml of pyrogen free water:

150 g of polyvinylpyrrolidone (K-value 11—14),

2.5 g of sodium formaldehydesulfoxylate,

8.7 g of magnesium oxide,

113.4 g of oxytetracycline hydrochloride, and

23.8 g of ethanolamine.

The volume was made up to 500 ml with pyrogen free water. The solution obtained contained 200 mg of oxytetracycline per ml. The pH of the solution was 9.0 and the viscosity at 22°C was 650 mPas.

In the next example the preparation is described, by way of comparison, of a solution with polyvinylpyrrolidone with a higher molecular weight ($K_{17}$).

Example 15

Preparation of a 10% oxytetracycline-solution with 25% of polyvinylpyrrolidone (K-value 15—17)

Under a nitrogen atmosphere the following was added successively to 200 ml of pyrogen free water:

125 g of polyvinylpyrrolidone (K-value 15—17, mean molecular weight 10,000),

3 g of sodium formaldehydesulfoxylate,

4.34 g of magnesium oxide,

11 g of ethanolamine and a solution of 56.7 g of oxytetracycline hydrochloride in 100 ml of pyrogen free water.

After stirring for some time, a clear solution was formed which was made up to 500 ml with pyrogen free water. The solution obtained contained 100 mg of oxytetracycline per ml and had a pH of 8.7 and a viscosity of 156 m Pas at 22°C. This solution was very difficult to filter through a sterilizing filter. The stability at 50°C was about 6 weeks and at 37°C more than half a year.

The preparation of Example 4 was administered intramuscularly to piglets and calves (groups of six animals). In the following tables the average serum concentrations obtained are shown.

TABLE I

Piglets; body weight ca. 24 kg, dose 8 mg/kg

| Hours post treatment | Oxytetracycline concentration (g/ml) |
|:---:|:---:|
| 1 | 3.0 |
| 3 | 2.9 |
| 5 | 3.1 |
| 7 | 2.4 |
| 14 | 0.97 |
| 24 | 0.39 |
| 28 | 0.31 |
| 31 | 0.19 |
| 48 | 0.008 |

TABLE II

Calves; body weight ca. 190 kg; dose 8 mg/kg

| Hours post treatment | Oxytetracycline concentration (g/ml) |
|:---:|:---:|
| 1 | 1.7 |
| 3 | 2.4 |
| 5 | 2.1 |
| 7 | 2.5 |
| 12 | 1.9 |
| 24 | 0.85 |
| 28 | 0.55 |
| 31 | 0.41 |
| 48 | 0.13 |
| 55 | 0.10 |

**0 125 725**

TABLE III

Piglets; body weight ca. 24 kg, dose 20 mg/kg

| Hours post treatment | Oxytetracycline concentration (g/ml) |
|---|---|
| 1 | 5.1 |
| 3 | 5.5 |
| 5 | 5.2 |
| 7 | 4.1 |
| 12 | 3.0 |
| 24 | 1.2 |
| 28 | 0.76 |
| 32 | 0.64 |
| 48 | 0.18 |
| 56 | 0.12 |

TABLE IV

Calves; body weight ca. 175 kg; dose 20 mg/kg

| Hours post treatment | Oxytetracycline concentration (g/ml) |
|---|---|
| 1 | 4.8 |
| 4 | 5.6 |
| 5 | 4.9 |
| 7 | 5.1 |
| 12 | 4.7 |
| 24 | 1.6 |
| 28 | 1.2 |
| 31 | 0.90 |
| 48 | 0.26 |
| 55 | 0.19 |
| 78 | 0.06 |

**Claims for the Contracting States: BE, CH, DE, FR, GB, IT, LI, LU, NL, SE**

1. Aqueous injectable solutions containing oxytetracycline, a magnesium compound and polyvinylpyrrolidone as the solubilizing agent, with a pH of 8—9.5, characterized in that the polyvinylpyrrolidone has a molecular weight between 500 and 10,000, and is present in a concentration of at least 18% (W/V), and the oxytetracycline is present in a concentration of at least 10% (W/V).

2. Aqueous injectable solutions according to claim 1, characterized in that the polyvinylpyrrolidone has a molecular weight between 1000 and 5000.

3. Process for the preparation of injectable oxytetracycline solutions which comprises dissolving in water oxytetracycline as a salt or a base with an appropriate amount of a magnesium compound and polyvinylpyrrolidone and bringing the pH in the range of 8.0 to 9.5, characterized in that the

7

polyvinylpyrrolidone has a molecular weight between 500 and 10,000, and a final concentration of at least 18% (W/V), and the oxytetracycline is present in a final concentration of at least 10% (W/V).

4. Process according to claim 3, characterized in that the polyvinylpyrrolidone has a molecular weight between 1000 and 5000.

**Claims for the Contracting State: AT**

1. Process for the preparation of injectable oxytetracycline solutions which comprises dissolving in water oxytetracycline as a salt or a base with an appropriate amount of a magnesium compound and polyvinylpyrrolidone and bringing the pH in the range of 8.0 to 9.5, characterized in that the polyvinylpyrrolidone has a molecular weight between 500 and 10,000, and a final concentration of at least 18% (W/V), and the oxytetracycline is present in a final concentration of at least 10% (W/V).

2. Process according to claim 1, characterized in that the polyvinylpyrrolidone has a molecular weight between 1000 and 5000.

**Patentansprüche für die Vertragsstaaten: BE, CH, DE, FR, GB, IT, LI, LU, NL, SE**

1. Wässrige injizierbare Lösungen, die Oxytetracyclin, eine Magnesiumverbindung und Polyvinylpyrrolidon als Lösungsvermittler enthalten, mit einem pH von 8 bis 9,5, dadurch gekennzeichnet, dass das Polyvinylpyrrolidon ein Molekulargewicht zwischen 500 und 10 000 hat und in einer Konzentration von mindestens 18% (Gewicht/Volumen) vorliegt und das Oxytetracyclin in einer Konzentration von mindestens 10% (Gewicht/Volumen) vorliegt.

2. Wässrige injizierbare Lösungen nach Anspruch 1, dadurch gekennzeichnet, dass das Polyvinylpyrrolidon ein Molekulargewicht zwischen 1000 und 5000 hat.

3. Verfahren zur Herstellung von injizierbaren Oxytetracyclinlösungen, das das Auflösen von Oxytetracyclin als Salz oder Base mit einer geeigneten Menge einer Magnesiumverbindung und Polyvinylpyrrolidon in Wasser und das Bringen des pH in den Bereich von 8,0 bis 9,5 umfasst, dadurch gekennzeichnet, dass das Polyvinylpyrrolidon ein Molekulargewicht zwischen 500 und 10 000 und eine Endkonzentration von mindestens 18% (Gewicht/Volumen) hat und das Oxytetracyclin in einer Endkonzentration von mindestens 10% (Gewicht/Volumen) vorhanden ist.

4. Verfahren nach Anspruch 3, dadurch gekennzeichnet, dass das Polyvinylpyrrolidon ein Molekulargewicht zwischen 1000 und 5000 hat.

**Patentansprüche für den Vertragsstaat: AT**

1. Verfahren zur Herstellung von injizierbaren Oxytetracyclinlösungen, das das Auflösen von Oxytetracyclin als Salz oder Base mit einer geeigneten Menge einer Magnesiumverbindung und Polyvinylpyrrolidon in Wasser und das Bringen des pH in den Bereich von 8,0 bis 9,5 umfasst, dadurch gekennzeichnet, dass das Polyvinylpyrrolidon ein Molekulargewicht zwischen 500 und 10 000 und eine Endkonzentration von mindestens 18% (Gewicht/Volumen) hat und das Oxytetracyclin in einer Endkonzentration von mindestens 10% (Gewicht/Volumen) vorhanden ist.

2. Verfahren nach Anspruch 1, dadurch gekennzeichnet, dass das Polyvinylpyrrolidon ein Molekulargewicht zwischen 1000 und 5000 hat.

**Revendications pour les Etats Contractants: BE, CH, DE, FR, GB, IT, LI, LU, NL, SE**

1. Solutions aqueuses injectables contenant de l'oxytétracycline, un composé du magnésium et de la polyvinylpyrrolidone comme agent solubilisant et ayant un pH de 8—9,5, caractérisées en ce que la polyvinylpyrrolidone a une masse moléculaire entre 500 et 10.000 et est présente en une concentration d'au moins 18% (p/v) et l'oxytétracycline est présente en une concentration d'au moins 10% (p/v).

2. Solutions aqueuses injectables suivant la revendication 1, caractérisées en ce que la polyvinylpyrrolidone a une masse moléculaire entre 1.000 et 5.000.

3. Procédé de préparation de solutions injectables d'oxytétracycline, qui comprend la dissolution, dans de l'eau, d'oxytétracycline à l'état de sel ou de base avec une quantité appropriée d'un composé du magnésium et de polyvinylpyrrolidone et l'ajustement du pH dans l'intervalle de 8,0 à 9,5, caractérisé en ce que la polyvinylpyrrolidone a une masse moléculaire entre 500 et 10.000 et une concentration finale d'au moins 18% (p/v) et l'oxytétracycline est présente en une concentration finale d'au moins 10% (p/v).

4. Procédé suivant la revendication 3, caractérisé en ce que la polyvinylpyrrolidone a une masse moléculaire entre 1.000 et 5.000.

**Revendications pour l'Etat Contractant: AT**

1. Procédé de préparation de solutions injectables d'oxytétracycline, qui comprend la dissolution, dans de l'eau, d'oxytétracycline à l'état de sel ou de base avec une quantité appropriée d'un composé du magnésium et de polyvinylpyrrolidone et l'ajustement du pH dans l'intervalle de 8,0 à 9,5, caractérisé en ce que la polyvinylpyrrolidone a une masse moléculaire entre 500 et 10.000 et une concentration finale d'au moins 18% (p/v) et l'oxytétracycline est présente en une concentration finale d'au moins 10% (p/v).

2. Procédé suivant la revendication 1, caractérisé en ce que la polyvinylpyrrolidone a une masse moléculaire entre 1.000 et 5.000.